# DEMANDE DE BREVET EUROPEEN

(11) **EP 2 412 337 A1**
(43) Date de publication de la demande: **01.02.2012**
(21) Numéro de dépôt: 11175244.0
(22) Date de dépôt: 25.07.2011
(51) Int. Cl.: A61F 2/16

(54) **Implant intraoculaire a éléments d'accrochage.**

(30) Priorité: 27.07.2010 FR 1056166
(71) Demandeur: Sourdille, Philippe, 86800 Liniers (FR)
(72) Inventeur: Sourdille, Philippe, 86800 Liniers (FR)
(74) Mandataire: Cabinet Plasseraud

(57) **Abrégé**

Implant intraoculaire (11) destiné à une implantation dans l'oeil (1) après une capsulorhexie de la capsule antérieure, comprenant une lentille (12) unique s'étendant dans un plan médian (13) et présentant un axe optique (14) normal au plan médian. L'implant comprend en outre une partie haptique (15) présentant des moyens d'immobilisation de la lentille (12) dans le sac capsulaire (4) de l'oeil. La partie haptique comprend une pluralité d'éléments de centrage/accrochage (27) et un cercle de référence (24) le long duquel s'étend un fond de centrage (18) de chacun des éléments de centrage/accrochage. Le cercle de référence (24) est parallèle et à distance axiale du plan médian (13) de la lentille (12). Les éléments de centrage/accrochage (23) présentent chacun un guide intérieur (19) et un guide extérieur (17) s'étendant radialement au-delà du fond de centrage (18) et sont destinés à entourer la capsule antérieure (7) respectivement à l'intérieur et à l'extérieur du sac capsulaire (4), le pourtour (10) de l'orifice de la capsule antérieure obtenu par capsulorhexie venant en appui sur les fonds de centrage (18).

## Description

### DOMAINE DE L'INVENTION

L'invention concerne le domaine des implants intraoculaires. Ces implants sont destinés à remplacer ou à corriger la fonction optique du cristallin. Ils comprennent une partie optique et des anses latérales de fixation appelées partie haptique.

L'invention concerne en particulier un implant intraoculaire destiné à une implantation dans l'oeil après une capsulorhexie de la capsule antérieure, notamment lorsque la capsule postérieure du sac cristallin est saine.

### ETAT DE LA TECHNIQUE ANTERIEURE

De tels implants sont utilisés, par exemple, pour le traitement de la cataracte. Ils sont insérés dans le sac cristallinien après ablation du matériau cristallinien. Le sac cristallinien est composé de deux calottes sphériques reliées entre elles appelées capsule antérieure et capsule postérieure. Le sac cristallinien est également appelé sac capsulaire. Avant insertion de l'implant, le chirurgien découpe un orifice généralement circulaire dans la capsule antérieure qui est située du côté de la cornée. Cette opération est appelée « capsulorhexie » et l'orifice réalisé est appelé « capsulorhexis » dont le diamètre varie de 4 à 7 mm.

La partie optique de tels implants pour oeil humain est généralement une lentille circulaire d'un diamètre de 5,5 mm à 7 mm.

La plupart des implants insérés dans le sac cristallinien prennent appui sur le diamètre de raccordement entre les capsules antérieure et postérieure appelé diamètre équatorial du cristallin. Or, le diamètre équatorial d'un individu humain adulte susceptible d'être opéré de la cataracte peut varier de 8,2 mm à 10,8 mm. Une des difficultés des implants intra cristalliniens est d'être compatibles avec les diamètres des yeux humains.

Par ailleurs, le document US 5 476 512 décrit un implant intraoculaire pour une implantation dans la chambre postérieure de l'oeil après une rupture de la capsule postérieure du sac cristallinien. Le document US2006/047339 décrit un collier de fixation de lentille qui comprend une gorge dans laquelle se loge le bord du capsulorhexis. La lentille est dans l'alignement de la capsule du sac cristallinien où ladite lentille est attachée. Toutes les haptiques décrites rejoingnent le diamètre équatorial du sac cristallinien.

### OBJET ET RESUME DE L'INVENTION

L'invention propose un implant intraoculaire qui répond à ce besoin en améliorant la compatibilité de l'implant avec une gamme plus large d'yeux humains.

Selon un mode de réalisation, l'implant intraoculaire destiné à une implantation dans l'oeil après une capsulorhexie de la capsule antérieure, comprenant :
- une lentille unique s'étendant dans un plan médian et présentant un axe optique normal au plan médian ainsi qu'une face postérieure destinée à être plaquée sur la capsule postérieure,
- l'implant comprenant en outre une partie haptique comprenant des guides intérieurs reliés à la lentille par un ou plusieurs bras de raccordement flexibles,
l'implant présentant des moyens d'immobilisation de la lentille dans le sac capsulaire de l'oeil,

Selon l'invention, les moyens d'immobilisation comprennent lesdits guides intérieurs, lesdits bras flexibles et au moins une portion de la face postérieure de la lentille, et sont conçus pour tendre la capsule antérieure et la capsule postérieure une fois que l'implant est en position. La partie haptique comprend une pluralité d'éléments de centrage/accrochage et un cercle de référence le long duquel s'étend un fond de centrage de chacun des éléments de centrage/accrochage, le cercle de référence étant parallèle et à distance axiale du plan médian de la lentille. Les éléments de centrage/accrochage présentent chacun un desdits guides intérieurs et un guide extérieur s'étendant radialement au-delà du fond de centrage et sont destinés à entourer la capsule antérieure respectivement à l'intérieur et à l'extérieur du sac capsulaire, le pourtour de l'orifice de la capsule antérieure obtenu par capsulorhexie venant en appui sur les fonds de centrage.

On comprend qu'un tel implant permet à la fois de sécuriser le centrage de l'implant, indépendamment des fluctuations du diamètre équatoriale du sac cristallinien, et de réduire le risque d'opacification secondaire.

En effet, les éléments de centrage/accrochage sont centrés par le diamètre fabriqué par le chirurgien lors de la capsulorhexie de la capsule antérieure. Cela permet de s'affranchir totalement de la variation de diamètres d'yeux humains d'un individu à l'autre et de la variation dans le temps du diamètre du cristallin due à la cicatrisation post opératoire. L'inventeur s'est en effet rendu compte que de gros progrès ont été réalisés dans les procédés opératoires de capsulorhexie de la capsule antérieure. Le chirurgien peut utiliser par exemple des anneaux de guidage pour obtenir un capsulorhexis bien centré et de dimensions stables d'une opération à l'autre. De nouvelles techniques de capsulorhexie par laser femtoseconde permettent également une bonne régularité de diamètre et un bon centrage. L'inventeur a trouvé qu'il devenait dorénavant possible de se centrer sur le diamètre capsulorhexis.

De plus, la tension exercée par les bras flexibles entre les guides intérieurs en appui du la capsule antérieure et au moins une portion de la face postérieure en appui sur la capsule postérieure permet de maintenir écarté l'une de l'autre la capsule antérieure et la capsule postérieure. Cela réduit fortement la migration des cellules germinatives qui se situes initialement dans la zone équatoriale du sac capsulaire. Cela réduit les risques d'opacification post opératoire de la capsule postérieure.

Un autre avantage d'un tel implant est de conserver la capsule postérieure lorsqu'elle est saine. Cela permet d'éviter toute contamination de la chambre postérieure de l'oeil très sensible lors de la pose de l'implant intraoculaire.

Selon un mode de réalisation, chaque bras de raccordement s'étend jusqu'à une distance de l'axe optique conçue pour qu'une fois implanté dans l'oeil, l'implant reste à une distance radiale prédéterminée non nulle du diamètre équatorial du sac cristallinien. Le fait que l'haptique ne ouche pas la zone où se trouve initialement les cellules germinatives permet de ralentir encore leur migration.

Avantageusement, le guide extérieur, le fond de centrage et le guide intérieur de chacun des éléments de centrage/accrochage sont de formes symétriques par rapport à un même plan méridien de l'élément de centrage/accrochage. On appelle plan méridien tout plan contenant l'axe optique de la lentille.

Avantageusement, le guide extérieur présente une extrémité distale libre et un arrondi en regard du guide intérieur. La distance axiale entre l'extrémité distale et le guide intérieur en regard est inférieure à l'épaisseur moyenne d'une capsule antérieure d'oeil humain, de façon à constituer un moyen de clipsage de la capsule antérieure.

Le fait que l'implant soit solidement clipsé sur la capsule antérieure confère une grande sécurité en cas de ré-intervention sur l'oeil ainsi implanté. En effet, il peut arriver que des cellules migrent entre la lentille et la capsule postérieure et opacifient cette capsule. Des opérations de récupération de cette opacification secondaire peuvent être entreprises pour découper par un laser traversant l'implant déjà installé, la partie de la capsule postérieure située au centre de manière juste nécessaire à restaurer l'acuité visuelle. La périphérie de la face postérieure de la lentille peut continuer à contribuer à l'immobilisation de l'implant dans l'oeil. Le fait que l'implant soit solidement clipsé et centré sur la capsule antérieure et plaqué sur la capsule postérieure réduit les risques de déplacement de l'implant après ouverture de la capsule postérieure.

La flexibilité des bras de raccordement facilite aussi l'insertion de l'implant en permettant d'étendre les bras pour introduire l'implant dans un dispositif d'insertion dans le sac cristallinien.

Avantageusement, la partie haptique est constituée par une pluralité de bras d'accrochages, constitués chacun par l'un des éléments de centrage/accrochage et par un unique bras de raccordement à la lentille, lui aussi de forme symétrique par rapport au plan méridien de l'élément de centrage/accrochage.

Selon un mode de réalisation, la lentille présente un bord latéral cylindrique et une face postérieure destinée à être plaquée contre la capsule postérieure de l'oeil, le bord cylindrique et la face postérieure étant raccordés par une arrête vive.

De tels implants sont connus sous l'appellation « d'implants à bords carrés ». L'arrête vive, ou bord carré, constitue un obstacle à la propagation des cellules germinatives le long le long de la capsule postérieure. L'invention est particulièrement avantageuse pour de tels implants. En effet, la tendance actuelle est de réduire l'épaisseur de la lentille et donc de réduire également la hauteur axiale du bord cylindrique. D'autre part, les techniques actuelles de polissage de l'implant risquent d'augmenter le rayon de courbure du bord carré, diminuant d'autant l'effet d'arrêt de la migration cellulaire. L'inventeur s'est rendu compte que le rayon de courbure de l'arrête vive n'est pas le seul paramètre à prendre en compte pour l'efficacité de la barrière à la propagation des cellules. L'inventeur a découvert que la pression de plaquage de la périphérie de la lentille contre la capsule postérieure est également déterminante. Ainsi, la combinaison des éléments du centrage/accrochage avec des bras de raccordement flexibles et des « bords carrés » permet d'améliorer l'efficacité de ces derniers même dans le cas de lentilles plus minces. En effet, le diamètre du capsulorhexis est très voisin de celui de la lentille. Donc, en prenant appui sur la capsule antérieure, les bras de raccordement exercent un effort de pression sensiblement au-dessus desdits « bords carrés ». On comprend que les implants prenant appui sur le diamètre équatorial du cristallin sont moins bien plaqués contre la capsule postérieure que ceux de l'invention.

Avantageusement, le bord latéral cylindrique s'étend axialement entre 150 et 300 µm. L'avantage de l'invention évoqué plus haut est particulièrement manifeste pour de tels implants à lentille amincie.

Avantageusement, chaque bras de raccordement est relié à une face antérieure de la lentille située du même côté du plan médian que le cercle de référence. Cela permet que la face postérieure et le bord latéral de la partie haptique ne soit pas modifiée par la présence des bras. Cela est particulièrement avantageux dans le cas d'implants dits « à bords carrés ».

Avantageusement, chaque bras de raccordement est relié à une zone périphérique de la lentille. Cela permet d'éviter que les bras modifient le comportement optique de l'implant.

Avantageusement, chaque bras de raccordement s'étend à moins de 4,2 mm de l'axe optique. Cela permet d'éviter que les bras de raccordement, à l'état implantés, ne soient en contact avec le diamètre équatorial du sac capsulaire qui est le lieu où se trouvent les sources de cellules germinatives responsables de l'opacification du cristallin.

Selon un mode de réalisation, les éléments d'accrochage présentent une section dans un plan méridien ayant la forme d'un U ou d'un V, le guide extérieur d'étendent sur une distance radiale de 0.6 à 2 mm.

Selon un mode de réalisation, la lentille et la partie haptique forment un ensemble monobloc en matériau synthétique hydrophile.

### BREVE DESCRIPTION DES FIGURES

D'autres caractéristiques et avantages de l'invention apparaîtront à la lecture de la description qui suit de plusieurs modes de réalisation de l'invention, donnés à titre d'exemples non limitatifs. La description se réfère aux dessins annexés sur lesquels :
- la figure 1 est une vue latérale d'un premier mode de réalisation d'implant intraoculaire selon la flèche F de la figure 2, et son implantation dans un oeil vu en coupe,
- la figure 2 est une vue de dessus du premier mode de réalisation,
- la figure 3 est une vue de dessus d'un deuxième mode de réalisation,
- la figure 4 est une vue de dessus d'un troisième mode de réalisation, et
- la figure 5 est une vue de dessus d'un quatrième mode de réalisation.

### DESCRIPTION DETAILLEE

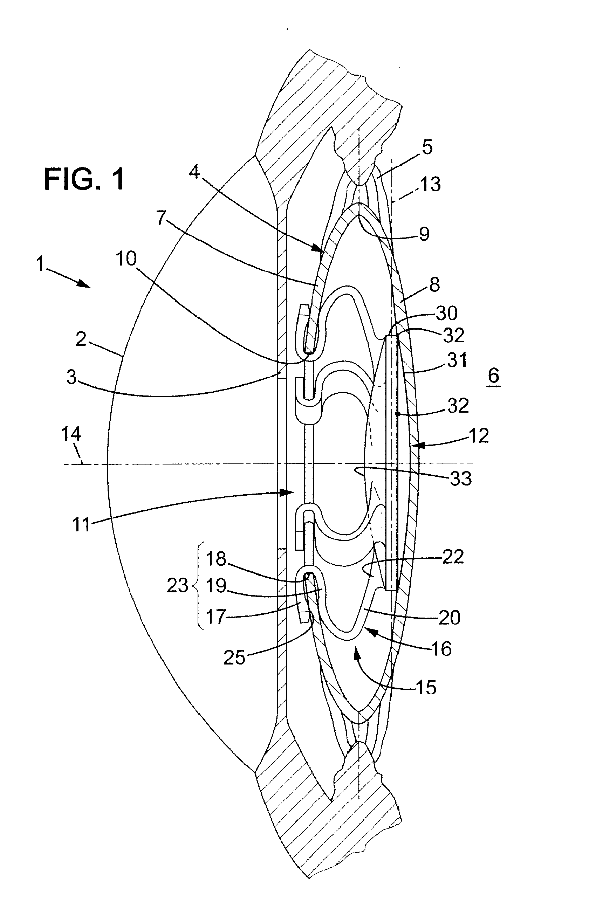

Comme illustré en Figure 1, l'oeil 1 comprend une cornée 2, un iris 3, un sac cristallinien 4 relié à l'oeil par une zonule 5, et une chambre postérieure 6 de l'oeil 1 située à l'arrière du sac cristallinien 4. Le sac cristallinien 4 est constitué d'une capsule antérieure 7 du côté de l'iris 3 et d'une capsule postérieure 8, hémisphériques toutes les deux et reliées en un diamètre équatorial 9.

L'oeil 1 est représenté après une opération de phacoémulsification au cours de laquelle le matériau cristallinien situé dans le sac cristallinien 4 a été détruit et retiré. De plus, un orifice circulaire a été ménagé dans la capsule antérieure 7 par une opération appelée capsulorhexie. Cet orifice présente un pourtour 10 appelé capsulorhexis 10.

Un implant 11 est constitué par une lentille 12 monobloc circulaire et s'étendant symétriquement le long d'un plan médian 13. La lentille 12 présente un axe optique 14 perpendiculaire au plan médian 13.

L'implant présente une partie haptique 15 constituée par quatre bras d'accrochage 16. Chacun des bras d'accrochage 16 est constitué par un guide extérieur 17, un fond de centrage 18, un guide intérieur 19 et un bras de raccordement 20 lequel est fixé sur une partie périphérique 21 de la lentille 12 par un pied 22. Le guide extérieur 17, le fond de centrage 18 et le guide intérieur 19 constituent un élément d'accrochage/centrage 23 ayant sensiblement la forme d'un U ouvert dans une direction radiale extérieure et entourant la capsule antérieure 7 en contournant le diamètre capsulorhexis 10. Le bras de raccordement 20 présente une forme en C, dans le prolongement du guide intérieur 19 et dont l'ouverture est dirigée vers l'axe optique 14.

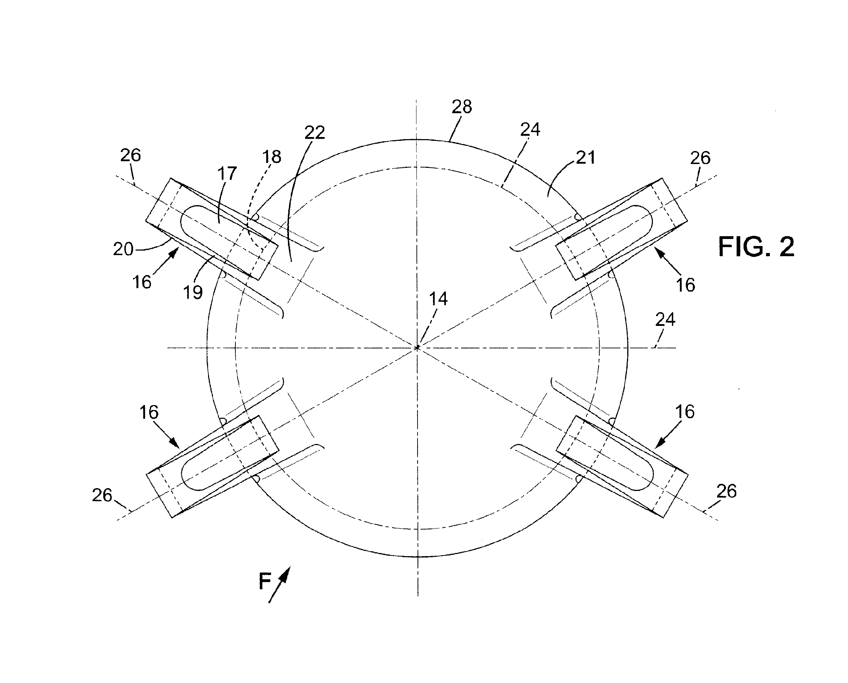

On comprend que chacun des fonds 18 de centrage sont à l'état libre avant implantation dans l'oeil 1, situé le long d'un cercle de référence 24 (visible en figure 2) parallèle et à distance du plan médian 13 de manière à correspondre au diamètre capsulorhexis 10 une fois implanté.

Le guide extérieur 17 présente, sur sa face en regard avec le guide intérieur 19, un arrondi 25 formant un bourrelet de clipsage.

La lentille 12 est une lentille de type « à bords carrés » en ce sens qu'elle présente un rebord latéral 30 cylindrique s'étendant axialement et présentant par rapport à une face postérieure 31 partiellement sphérique, une arrête vive de raccordement 32 faisant obstacle à la propagation des cellules germinatives en provenance du diamètre équatorial 9 afin d'éviter une propagation entre la face postérieure 31 de la lentille 12 et la capsule postérieure 8.

Chacun des pieds 22 des bras d'accrochage 16 s'étend sur la face antérieure 33 de la lentille 12 de manière à ne pas affecter la géométrie de l'arrête de raccordement 32.

Lorsque l'implant 11 est introduit dans le sac capsulaire 4, les bras de raccordement 20 sont légèrement comprimés axialement de manière à tendre la capsule antérieure 7 et la capsule postérieure 8 et à renforcer l'effort de plaquage de l'arrête vive 32 contre la capsule postérieure 8.

Comme illustré en Figure 2, chacun des quatre bras d'accrochage 16 s'étendent symétriquement par rapport à quatre demi plans méridiens 26 passant par l'axe optique 14 et incliné chacun de 30° par rapport à un plan de symétrie 27 par rapport auquel la lentille 12 est pliée pour faciliter son insertion dans l'oeil grâce à une seringue d'injection. Lors du pliage autour du plan 27, les bras d'accrochage 16 qui sont flexibles s'étendent sur la droite et la gauche de la Figure 2 de manière à ne pas dépasser du diamètre extérieur 28 de la lentille 12.

Une fois implantés dans le sac capsulaire 4, chacun des bras d'accrochage 16 reprennent leur position de repos telle qu'illustrée aux Figures 1 et 2. Les fonds de centrage 18 de chacun des éléments de centrage/accrochage 23 sont situés le long du cercle de référence 24.

Une fois implantés dans l'oeil, le chirurgien peut, avec un outil, attraper chacun des bras d'accrochage et l'amener chacun vers l'axe optique 14 de manière que le guide extérieur 17 puisse ressortir du sac capsulaire 4 et que le fond de centrage 18 vienne alors en appui sur le pourtour 10 de l'orifice obtenu par capsulorhexie.

Chacun des bras d'accrochage 16 présente une largeur qui décroît progressivement de 9 mm à 5 mm de large au fur et à mesure du pied 22. L'épaisseur varie également de 1 mm à 0,6 mm. On comprend que de tels bras d'accrochage présentent une raideur s'opposant au déplacement axial de l'élément de centrage/ accrochage 23 inférieur à la raideur latérale s'opposant à un déplacement de ce même élément de centrage 23 dans un plan parallèle au plan médian 13. Une telle configuration permet à la fois un bon centrage de la lentille 12 par rapport au pourtour capsulorhexis 10 tout en permettant une légère tension axiale des chambres postérieure et antérieure 8, 7.

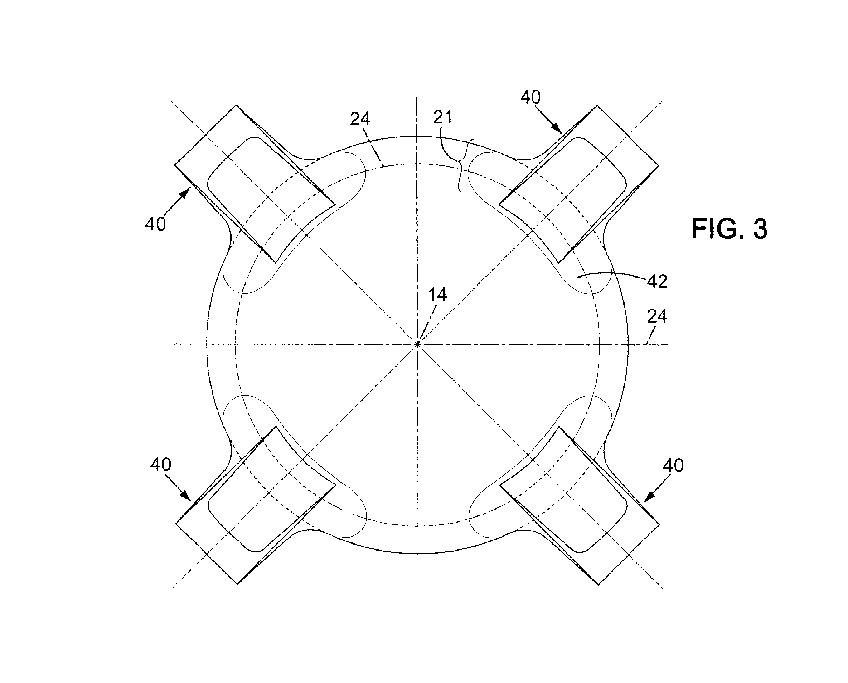

Comme illustré en Figure 3, un deuxième mode de réalisation comprend quatre bras d'accrochage 40 situés à 90° les uns des autres et présentant une grande largeur, par exemple de l'ordre de 14 mm. Cela vient renforcer la qualité du centrage de la lentille 12 par rapport au capsulorhexis 10.

Le pied de raccordement 42 du bras d'accrochage 40 s'étend latéralement de part et d'autre du plan méridien de manière à cantonner le pied de raccordement 42 le plus près possible de la zone périphérique 21 de la lentille 12.

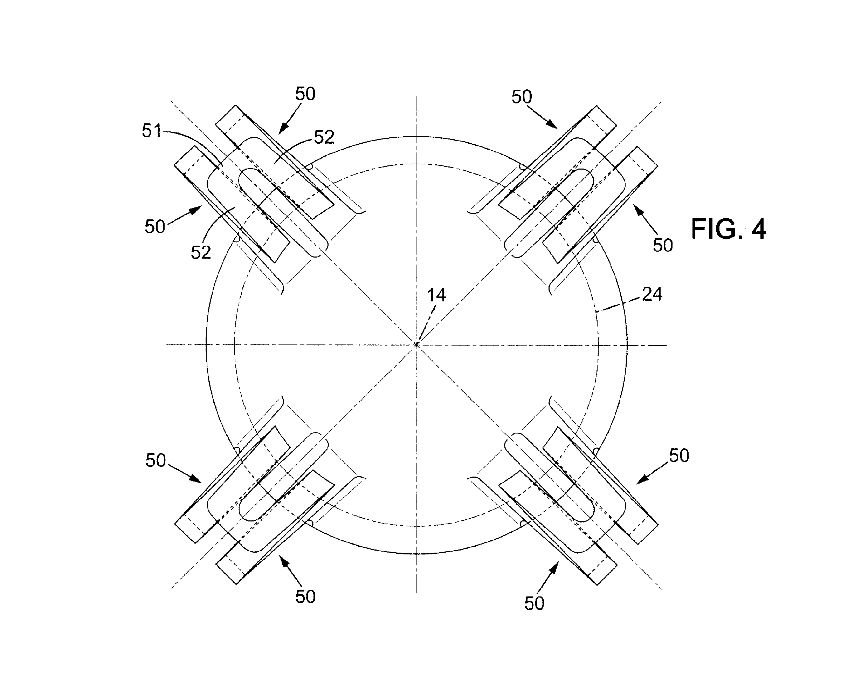

Comme illustré en Figure 4, un troisième mode de réalisation est constitué de plusieurs bras d'accrochage 50 jumelés entre eux, deux par deux, par un pont 51 de raccordement des guides extérieurs 52. Ce mode de réalisation présente à la fois des bras d'accrochage plus souples susceptibles de se plier davantage pour faciliter l'insertion dans le sac capsulaire tout en permettant au chirurgien de positionner simultanément deux bras d'accrochage 50 en raison du pont de raccordement 51.

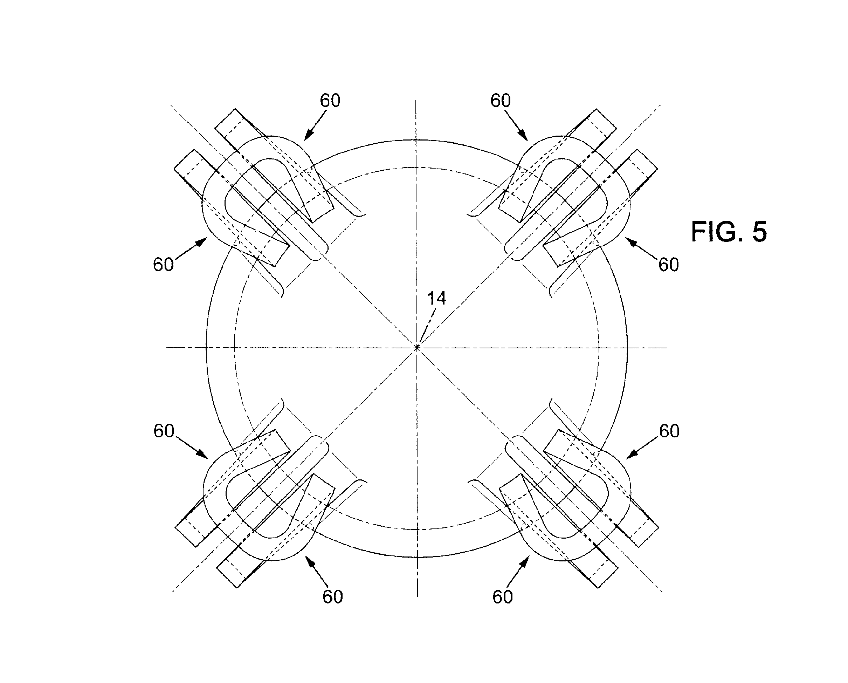

Comme illustré en Figure 5, les bras de raccordement 60 sont également jumelés deux par deux tout en étant encore plus souples.

On comprendra que les différents modes de réalisation évoqués peuvent être combinés entre eux, on peut par exemple faire varier la larguer de chacun des bras de raccordement ainsi que leur forme. De même, les bras d'accrochage 16, 50, 60 pourraient aussi être équipés de pied de raccordement élargis latéralement et réduit radialement.

Dans une autre variante, le nombre de bras d'accrochage 6, ou 40, ou 50 peut être par exemple de trois ou de six.

## Revendications

1. Implant intraoculaire (11) destiné à une implantation dans l'oeil (1) après une capsulorhexie de la capsule antérieure (7), comprenant :
- une lentille unique (12) s'étendant dans un plan médian (13) et présentant un axe optique (14) normal au plan médian ainsi qu'une face postérieure destinée à être plaquée sur la capsule postérieure,
- l'implant comprenant en outre une partie haptique (15) comprenant des guides intérieurs (19) reliés à la lentille (12) par un ou plusieurs bras de raccordement flexibles (20),
l'implant présentant des moyens d'immobilisation de la lentille (12) dans le sac capsulaire (4) de l'oeil,
**caractérisé en ce que** les moyens d'immobilisation comprennent lesdits guides intérieurs (19), lesdits bras flexibles et au moins une portion de la face postérieure (31) de la lentille, et sont conçus pour tendre la capsule antérieure (7) et la capsule postérieure (8) une fois que l'implant est en position,
**en ce que** la partie haptique (15) comprend une pluralité d'éléments de centrage/accrochage (23) et un cercle de référence (24) le long duquel s'étend un fond de centrage (18) de chacun des éléments de centrage/accrochage, le cercle de référence (24) étant parallèle et à distance axiale du plan médian (13) de la lentille (12);
et **en ce que** les éléments de centrage/accrochage (23) présentent chacun un desdits guides intérieurs (19) et un guide extérieur (17) s'étendant radialement au-delà du fond de centrage (18) et sont destinés à entourer la capsule antérieure (7) respectivement à l'intérieur et à l'extérieur du sac capsulaire (4), le pourtour (10) de l'orifice de la capsule antérieure obtenu par capsulorhexie venant en appui sur les fonds de centrage (18).

2. Implant selon la revendication 1, dans lequel chaque bras de raccordement s'étend jusqu'à une distance de l'axe optique conçue pour qu'une fois implanté dans l'oeii, l'implant reste à une distance radiale prédéterminée non nulle du diamètre équatorial du sac cristallinien.

3. Implant selon l'une des revendications précédentes, dans lequel le guide extérieur (17), le fond de centrage (18) et le guide intérieur (19) de chacun des éléments de centrage/accrochage (23) sont de formes symétriques par rapport à un même plan méridien (26) de l'élément de centrage/accrochage (23).

4. Implant selon l'une des revendications précédentes,, dans lequel le guide extérieur (17) présente une extrémité distale libre et un arrondi (25) en regard du guide intérieur (19), la distance axiale entre l'extrémité distale et le guide intérieur en regard étant inférieure à l'épaisseur moyenne d'une capsule antérieure d'oeil humain, de façon à constituer un moyen de clipsage de la capsule antérieure (7).

5. Implant selon l'une des revendications précédentes, dans lequel la partie haptique (15) est constituée par une pluralité de bras d'accrochage (16,40,50,60), constitués chacun par l'un des éléments de centrage/accrochage et par un unique bras de raccordement (20) à la lentille (12), lui aussi de forme symétrique par rapport au plan méridien (26) de l'élément de centrage/accrochage (23).

6. Implant selon l'une des revendications précédentes, dans lequel chaque bras de raccordement (20) est relié à une zone périphérique (21) de la lentille (12).

7. Implant selon l'une des revendications précédentes, dans lequel chaque bras de raccordement (20) est relié à une face antérieure (33) de la lentille (12) située du même coté du plan médian (13) que le cercle de référence (24).

8. Implant selon l'une des revendications précédentes, dans lequel chaque bras de raccordement (20) s'étend à moins de 4,2 mm de l'axe optique (14).

9. Implant selon l'une des revendications précédentes, dans lequel la lentille (12) présente un bord latéral (30) cylindrique et une face postérieure (31) destinée à être plaquée contre la capsule postérieure (8) de l'oeil, le bord cylindrique (30) et la face postérieure (31) étant raccordés par une arrête vive (32).

10. Implant selon la revendication 9, dans lequel le bord latéral cylindrique (30) s'étend axialement entre 150 et 300 µm.
